(19) Europäisches Patentamt
European Patent Office
Office européen des brevets

(11) Publication number: **0 452 697 B1**

(12) # EUROPEAN PATENT SPECIFICATION

(45) Date of publication of patent specification: **06.12.95**

(51) Int. Cl.[6]: **A61K 31/16**, A61K 47/12

(21) Application number: **91104502.9**

(22) Date of filing: **22.03.91**

(54) **Stabilizer for 4-ethyl-2-hydroxyimino5-nitro-3-hexenamide-containing preparation and stabilizing method therefor.**

(30) Priority: **28.03.90 JP 81790/90**
**10.08.90 JP 213051/90**

(43) Date of publication of application:
**23.10.91 Bulletin 91/43**

(45) Publication of the grant of the patent:
**06.12.95 Bulletin 95/49**

(84) Designated Contracting States:
**AT BE CH DE DK ES FR GB GR IT LI LU NL SE**

(56) References cited:
**EP-A- 0 113 106**
**FR-A- 2 333 494**

**PATENT ABSTRACTS OF JAPAN, vol. 9, no. 145 (C-287)[1868], 20th June 1985; & JP-A-60 028 923 (TEIJIN K.K.) 14-02-1985**

**PATENT ABSTRACTS OF JAPAN, vol. 8, no. 150 (C-233)[1587], 12th July 1984; & JP-A-59 055 828 (NITTO DENKI KOGYO K.K.) 31-03-1984**

**The Merck Index, 11th edition, 1989, pages 867 and 1093**

(73) Proprietor: **FUJISAWA PHARMACEUTICAL CO., LTD.**
**4-7, Doshomachi 3-chome**
**Chuo-ku**
**Osaka-shi**
**Osaka 541 (JP)**

(72) Inventor: **Ueda, Yoshio**
**3-5-204, Mikagenakamachi 1-chome,**
**Higashinada-ku**
**Kobe-shi,**
**Hyogo 658 (JP)**
Inventor: **Onishi, Norio**
**10, Sagatenryujikitatsukurimichicho**
**Ukyo-ku,**
**Kyoto-shi,**
**Kyoto 616 (JP)**
Inventor: **Yasumura, Mitsuru**
**5-37, Matsuzonocho**
**Nishinomiya-shi,**
**Hyogo 662 (JP)**
Inventor: **Okimoto,Kazuto. rm 106, Rohderu Crt Esta Gakuenmae**
**2-116-5, Gakuendaiwacho**
**Nara-shi,**
**Nara 631 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 452 697 B1**

**Martindale The Extra Pharmacapoeia, 28th edition, 1982, pages 1281-1282, 1287 and 1653-1657**

(74) Representative: **Türk, Gille, Hrabal, Leifert**
**Brucknerstrasse 20**
**D-40593 Düsseldorf (DE)**

## Description

BACKGROUND OF THE INVENTION

Field of the Invention

The present invention relates to a use of a stabilizer for 4-ethyl-2-hydroxyimino-5-nitro-3-hexenamide represented by the following chemical formula (I), in particular, (± )-(E)-4-ethyl-2-[(E)-hydroxyimino]-5-nitro-3-hexenamide or a salt thereof acceptable as drug or drugs containing as pharmacologically active ingredient the above compound, and a stabilizing method by the use thereof.

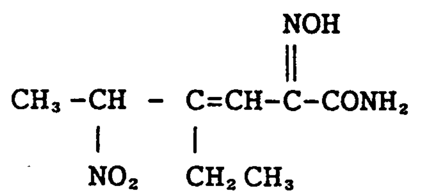

... (I)

Description of the Prior Art

It is well know from Japanese Laid-open Patent Publication No. 59-152366 that (E)-4-ethyl-2-hydroxyimino-5-nitro-3-hexenamide or its salt/s accepted as drug has pharmacological activity as a vasodilating drug, anti-thrombotic drug, drug for angina pectoris or the like and, it is disclosed in the aforementioned publication that this compound can be manufactured as drugs in form of tablet, capsule, pellet, suppository and the like and that various excipients can be used in the manufacture thereof. Through further studies it has been confirmed that the aforementioned compound, in particular, (±)-(E)-4-ethyl-2-[(E)-hydroxyimino]-5-nitro-3-hexenamide or a salt thereof has a excellent pharmacological activity. Since this compound is called FK409 by this applicant and about it clinical studies are now under way, the compound to be stabilized by the method of the present invention will hereinafter be represented by this name.

SUMMARY OF THE INVENTION

FK409 is poor in stability and when, for instance, it is left standing at 40 °C for 2 months, it is completely decomposed to a dark brown fused substance with its pharmacological activity lost. For manufacture of drugs in some of the aforementioned forms it has to be mixed with proper excipients but when mixed with an excipient, it is extremely poor in stability and the content of the active ingredient is markedly reduced when it is left standing for 1 month at 40 °C.

The present invention has been made for improvement in this respect and is aimed at provision of a use of a stabilizer effective for preventing decomposition of FK409 and having its pharmacological activity kept for a long period. Another object of the present invention is provision of a method of stabilizing FK409 or drugs containing it as the pharmacologically active ingredient.

JP-A-60-28923 discloses an organic acid (e.g. stearic acid, palmitic acid, citric acid, tartaric acid etc.) as a stabilizer in a pharmaceutical composition applicable to the oral mucosa.

FR-A-2 333 494 discloses a process for stabilising a solution of a derivate of pyrido(3, 2-a)phenoxazine in which ascorbic acid and isoascorbic acid is used as a stabilizer.

Martindale, The Extra Pharmacopoeia, 28th Edition, 1992, pages 1281-1282, 1287, and 1653-1657 mentions ascorbic acid and isoascorbic acid as antioxidants for pharmaceutical preparations.

DETAILED DESCRIPTION OF THE INVENTION

The stabilizer used according to the present invention is characterized in that it comprises one or more of organic acids selected from tartaric acid, stearic acid, ascorbic acid, erythorbic acid and malonic acid and the method of the invention is characterized in that stabilization is attained by mixing the aforementioned stabilizer in drugs containing FK409, and the stability of FK409 as a pharmacological ingredient is

markedly improved and FK409-containing drugs excelled in durability of activity are obtained. The present inventors studied various compounds to see their stabilizing effect, that is, to see if they are effective for preventing decomposition of FK409, and as a result discovered that compounds selected from organic acids selected from tartaric acid, stearic acid ascorbic acid, erythorbic acid and malonic acid have excellent stabilization effect. When the above exemplified polycarboxylic acid has an asymmetric carbon atom(s) such as tartaric acid, or malonic acid, each of D-form, L-form or racemic mixture may be used.

The salt of FK409 may be pharmaceutically acceptable salt including organic or inorganic salt.

As to the quantity of the aforementioned compound to be added as stabilizer, there is no limitation but it is preferred to be not less than 0.1 weight part, more preferably in a range of 0.3-5 weight part, of the quantity of FK409.

The drug composition of the present invention can be mixed with organic or inorganic carriers or excipients suited for external, oral or non-oral administration so as to be usable as solid, semisolid or liquid medical preparations containing the effective substance of the invention. The invented effective substance (ingredient) may be mixed with any of the ordinary, nontoxic carriers permitted for medical use and suited for preparation of tablets, pellets, capsules, suppositories, solutions, emulsions, suspensions and the like. As such carriers may be cited water, dextrose, lactose, gum arabic, gelatin, mannitol, starch paste, magnesium silicate, talc, corn starch, keratin, colloid silica, potato starch and urea in solid, semisolid or liquid form, being suited for drug manufacture, and auxiliaries, stabilizers, thickeners, colorants and aromatics are also usable. It is also possible to use preservatives or bacteriostats for stably maintaining the activity of the drug or its effective ingredient in any given form. The medical composition of the invention is also usable for manufacture of persistent drugs of various forms. It is also possible to use the aforementioned drugs as long lasting drugs of various forms.

FK409 is poor in stability when mixed with an excipient for drug manufacture and its activity is lost quickly when the mixture is kept in stock at a high temperature, but its decomposition is markedly prevented when one of the aforementioned compounds is added as stabilizer for the resultant preparation or drug to be highly improved in durability of pharmacological activity.

The FK409 content of a drug of the present invention may be determined properly with the stage of the disease the drug is prescribed for and its form, if the desired therapeutic effect could be attained.

In applying the drug of the invention to human being, it may be prepared in various forms suited for venous or muscular injection, cutaneous administration as in the case of plaster or suppository or oral administration. The dosage depends on the stage of disease and the age of the patient but, generally, the effective dose of FK409 is approx. 0.1-100 mg/kg a day and normal per-time dose is 10 mg, 50 mg, 100 mg or 250 mg on the average.

## Example

The composition and effect of the drug of the present invention will be specifically described below with reference to the cited example.

First the stability of of FK409 when it was kept in a capsule, when it was stored mixed with an excipient and when it was mixed with tartaric acid as stabilizer (the residual percentage of FK409) was studied. In the experiment, however, the individual samples were put in #1 bottles, the filled bottles were then sealed, kept at the predetermined temperature and upon lapse of the predetermined time, the residual percentage of FK409 was measured by the liquid chromatographic method and its proportion to the initial content was determined.

The compositions of the individual samples are shown in Table 1 and the results in Table 2.

Table 1

| | Recipe No. | 1 | 2 | 3 | 4 |
|---|---|---|---|---|---|
| Composition (mg) | FK409 | 3 | 3 | 3 | 3 |
| | Lactose | - | 87 | 84 | - |
| | D-Mannit | - | - | - | 84 |
| | DL-Tartaric acid | - | - | 3 | 3 |
| | Total | 3 | 90 | 90 | 90 |

Table 2

| | | | Residual percentage (%) | | | |
|---|---|---|---|---|---|---|
| | | | Recipe 1 | Recipe 2 | Recipe 3 | Recipe 4 |
| original powder of FK409 | | | 100 | 100 | 100 | 100 |
| Storage condition | 40℃ | 1 month | 0 | 0 | 95.3 | 98.6 |
| | | 3 months | - | - | 82.4 | 99.1 |
| | Room temp. | 6 months | 0 | 0 | 96.1 | 100 |

As seen from Tables 1 and 2, FK409, either in the form of original powder (recipe No. 1) or mixed with an excipient (recipe No.2), totally loses its pharmacological activity after storage for 1 month at 40 °C , but when a proper dose of tartaric acid is added (as stabilizer), decomposition of FK409 is markedly prevented and its residual percentage is largely increased.

Table 3 below is given to show the result of study about various compounds on their stabilizing effect on the original powder of FK409.

Table 3

| | Stabilizer dose(g) | Storage condition and residual (%) | | | |
|---|---|---|---|---|---|
| | | 40 °C | 50 °C | 60 °C | 60 °C |
| FK409 | | 1 month | 1 month | 18 days | 7 days |
| Original powder | - | 0 | 0 | 0 | 99.3 |
| DL-Tartaric acid | 1 | 97.2 | 98.0 | 88.1 | 99.0 |
| Stearic acid | 1 | 99.3 | 95.2 | - | - |
| Vitamin C | 1 | 97.1 | 98.3 | 87.8 | 99.0 |
| Erythorbic acid | 1 | 96.3 | 98.4 | 83.5 | 99.8 |
| DL-Malonic acid | 1 | 98.9 | 83.5 | 32.5 | 90.1 |
| DL-citric acid anhydride | 1 | 70.1 | 76.3 | - | - |
| The dose of stabilizer in g is per 1 g of the original powder of FK409. | | | | | |

As is apparent from Table 3, the compounds used in this experiment all have excellent stabilizing effect on FK409, DL-tartaric acid, stearic acid, vitamin C and erythorbic acid in particular.

The storage conditions in this experiment are rather severe ones, one month of storage at 50 °C being equivalent to storage at the room temperature for approx. 12 months and, this taken into consideration, the effect of this invention is truly outstanding.

Then, the stabilizing effect of tartaric acid on tablets with FK409 as active ingredient will be demonstrated.

The ingredients in the recipe shown Table 4 except only magnesium stearate were mixed, 40 ml of water was added to the mixture (approx. 135 g) and the wetted mixture was uniformly kneaded, vacuum dried and granulated. To the granules the prescribed amount of magnesium stearate was added and, after mixing, the mixture was made into tablets 7mm in diameter by the use of a tablet making machine.

40 tablets thus obtained were put in a #3 bottle, the filled sealed bottle was stored for the predetermined period at the room temperature (25 °C ) or 40 °C , and then the residual percentage of FK409 was studied.

The result was as shown in Table 5, and from the tabulated data it is apparent that tartaric acid has an excellent stabilizing effect on FK409 even when it is prepared in tablet form.

Table 4

| Recipe No. | | 1 | 2 | 3 | 4 |
|---|---|---|---|---|---|
| Composition (mg) | FK409 (Pharma. ingredient) | 10 | 40 | 10 | 40 |
| | DL-Tartaric acid (stabilizer) | 3 | 12 | 3 | 12 |
| | D-Mannit | 107.95 | 68.95 | 107.95 | 68.95 |
| | ECG 505 | 12 | 12 | - | - |
| | Ac-Di-So1 | - | - | 12 | 12 |
| | TC-5E | 1.35 | 1.35 | 1.35 | 1.35 |
| | Magnesium stearate | 0.7 | 0.7 | 0.7 | 0.7 |
| | Total | 135 | 135 | 135 | 135 |
| ECG 505: Carboxyl methyl cellulose calcium (disintegrator)<br>Ac-Di-So1: Crosslinked-carboxyl methyl cellulose-sodium (disintegrator)<br>TC-5E: Hydroxy propyl methyl cellulose (binder)<br>Magnesium stearate (lubricant) | | | | | |

Table 5

| | | | Residual percentage (%) | | | |
|---|---|---|---|---|---|---|
| | | | Recipe 1 | Recipe 2 | Recipe 3 | Recipe 4 |
| Initial | | | 100.0 | 100.0 | 100.0 | 100.0 |
| Storage condition | 40 °C | 1 month<br>6 months | 98.9<br>96.8 | 100.0<br>97.3 | 99.9<br>95.1 | 100.0<br>95.8 |
| | Room temp. | 1 month<br>6 months | 100.0<br>99.2 | 99.9<br>99.8 | 99.3<br>99.3 | 100.0<br>99.6 |

## Claims

**Claims for the following Contracting States : AT, BE, CH, LI, DE, DK, FR, GB, IT, LU, NL, SE**

1. Use of an organic acid selected from the group of tartaric acid, stearic acid, ascorbic acid, erythorbic acid and malonic acid as stabilizer for 4-ethyl-2-hydroxyimino-5-nitro-3-hexenamide represented by the following chemical formula (I) or a salt thereof acceptable as drug or drug containing as pharmacologically active ingredient a compound represented by the chemical formula (I) or a salt thereof acceptable as drug.

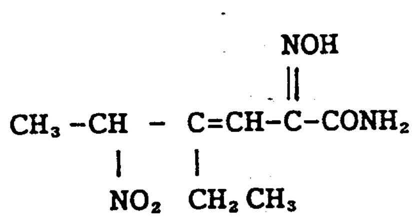

... (I)

2. Use according to claim 1 wherein said 4-ethyl-2-hydroxyimino-5-nitro-3-hexenamide is (± )-(E)-4-ethyl-2-[(E)-hydroxyimino]-5-nitro-3-hexenamide.

3. Use according to claim 1 or 2, wherein the quantity added of said stabilizer is not less than 0.1 weight part is mixed with 1 weight part of said compound represented by said chemical formula (I) or a salt thereof accepted as drug.

4. Use according to claim 3, wherein the quantity added of said stabilizer is 0.3-5 weight part.

**Claims for the following Contracting States : ES, GR**

1. A stabilizing method for a compound represented by the following chemical formula (I)

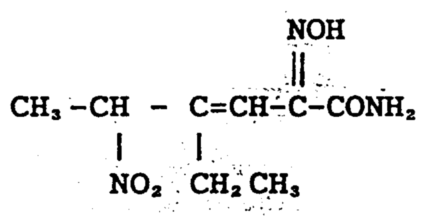

... (I)

or a salt thereof acceptable as a drug, wherein as stabilizer an organic acid selected from the group of tartaric acid, stearic acid, ascorbic acid, erythorbic acid and malonic acid is mixed with a compound represented by said chemical formula (I) or a salt thereof accepted as drug.

2. A stabilizing method according to claim 1, wherein said compound represented by the chemical formula (I) is (±)-(E)-4-ethyl-2-[(E)-hydroxyimino]-5-nitro-3-hexenamide.

3. A stabilizing method according to claim 1 wherein the quantity added of said stabilizer is not less than 0.1 weight part is mixed with 1 weight part of said compound represented by said chemical formula (I) or a salt thereof accepted as drug.

4. A stabilizing method according to claim 3 wherein the quantity added of said stabilizer is 0,3-5 weight part.

**Patentansprüche**
**Patentansprüche für folgende Vertragsstaaten : AT, BE, CH, LI, DE, DK, FR, GB, IT, LU, NL, SE**

1. Verwendung einer organischen Säure, ausgewählt aus der Gruppe aus Weinsäure, Stearinsäure, Ascorbinsäure, Isoascorbinsäure und Malonsäure als Stabilisierungsmittel für 4-Ethyl-2-hydroxyimino-5-nitro-3-hexenamid, dargestellt durch die folgende Formel (I), oder ein pharmazeutisch verträgliches Salz davon, oder für ein Arzneimittel, das als pharmakologisch wirksamen Inhaltsstoff die durch die chemische Formel (I) dargestellte Verbindung oder ein pharmazeutisch verträgliches Salz davon enthält.

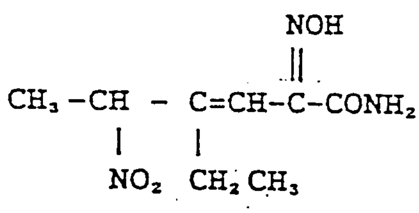

... (I)

2. Verwendung nach Anspruch 1, worin das 4-Ethyl-2-hydroxyimino-5-nitro-3-hexenamid das (±)-(E)-4-Ethyl-2-[(E)-hydroxyimino]-5-nitro-3-hexenamid ist.

3. Verwendung nach den Ansprüchen 1 oder 2, worin eine Menge von nicht weniger als 0,1 Gewichtsteilen des hinzugesetzten Stabilisierungsmittels mit 1 Gewichtsteil der durch die chemische Formel (I) dargestellten Verbindung oder deren pharmazeutisch verträgliches Salz vermischt wird.

4. Verwendung nach Anspruch 3, worin die Menge des hinzugesetzten Stabilisierungsmittels 0,3-5 Gewichtsteile beträgt.

**Patentansprüche für folgende Vertragsstaaten : ES, GR**

1. Verfahren zur Stabilisierung einer Verbindung der folgenden chemischen Formel (I)

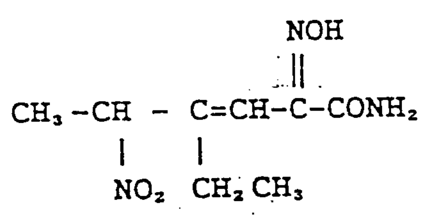

... (I)

worin als Stabilisierungsmittel eine organische Säure, ausgewählt aus der Gruppe aus Weinsäure, Stearinsäure, Ascorbinsäure, Isoascorbinsäure und Malonsäure mit einer durch die folgende Formel (I) dargestellten Verbindung, oder einem pharmazeutisch verträglichen Salz davon vermischt wird.

2. Verfahren zur Stabilisierung nach Anspruch 1, worin die durch die Formel (I) dargestellte Verbindung (±)-(E)-4-Ethyl-2-[(E)-hydroxyimino]-5-nitro-3-hexenamid ist.

3. Verfahren zur Stabilisierung nach Anspruch 1, worin eine Menge von nicht weniger als 0,1 Gewichtsteilen des hinzugesetzten Stabilisierungsmittels mit 1 Gewichtsteil der durch die chemische Formel (I) dargestellten Verbindung oder deren pharmazeutisch verträgliches Salz vermischt wird.

4. Verfahren zur Stabilisierung nach Anspruch 3, worin die Menge des Stabilisierungsmittels 0,3-5 Gewichtsteile beträgt.

**Revendications**
**Revendications pour les Etats contractants suivants: AT, BE, CH, LI, DE, DK, FR, GB, IT, LU, NL, SE**

1. Utilisation d'un acide organique choisi dans le groupe constitué de l'acide tartrique, de l'acide stéarique, de l'acide ascorbique, de l'acide érythorbique, et de l'acide malonique comme agent de stabilisation pour le 4-éthyl-2-hydroxyimino-5-nitro-3-hexèneamide représenté par la formule chimique (I) suivante ou un de ses sels acceptable comme médicament ou un médicament contenant à titre d'ingrédient pharmaceutiquement actif un composé représenté par la formule chimique (I) ou un de ses sels acceptable comme médicament

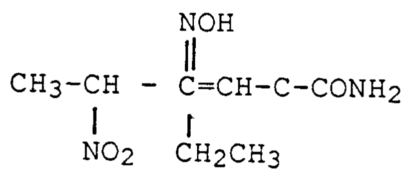
...(I)

2. Utilisation selon la revendication 1, dans laquelle ledit 4-éthyl-2-hydroxyimino-5-nitro-3-hexèneamide est le (±)-(E)-4-éthyl-2-[(E)-hydroxyimino]-5-nitro-3-hexèneamide.

3. Utilisation selon la revendication 1 ou la revendication 2, dans laquelle la quantité ajoutée dudit agent de stabilisation n'est pas inférieure à 0,1 partie en poids et est mélangée avec 1 partie en poids dudit composé représenté par ladite formule chimique (I) ou d'un de ses sels accepté comme médicament.

4. Utilisation selon la revendication 3, dans laquelle la quantité ajoutée dudit agent de stabilisation est de 0,3 à 5 parties en poids.

**Revendications pour les Etats contractants suivants : ES, GR**

1. Procédé de stabilisation pour un composé représenté par la formule chimique (I) suivante :

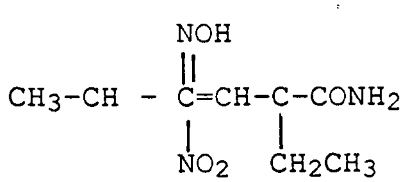
...(I)

ou un de ses sels acceptable comme médicament, dans lequel, à titre d'agent de stabilisation, on mélange un acide organique choisi dans le groupe constitué de l'acide tartrique, de l'acide stéarique, de l'acide ascorbique, de l'acide érythorbique, et de l'acide malonique, avec un composé représenté par ladite formule chimique (I) ou un de ses sels accepté comme médicament.

2. Procédé de stabilisation selon la revendication 1, dans lequel ledit composé représenté par la formule chimique (I) est le (±)-(E)-4-éthyl-2-[(E)-hydroxyimino]-5-nitro-3-hexèneamide.

3. Procédé de stabilisation selon la revendication 1, dans lequel la quantité ajoutée dudit agent de stabilisation n'est pas inférieure à 0,1 partie en poids et est mélangée avec 1 partie en poids dudit

composé représenté par ladite formule chimique (I) ou d'un de ses sels accepté comme médicament.

4. Procédé de stabilisation selon la revendication 3, dans lequel la quantité ajoutée dudit agent de stabilisation est de 0,3 à 5 parties en poids.